# EUROPEAN PATENT APPLICATION

(11) **EP 4 643 795 A1**
(43) Date of publication of application: **05.11.2025**
(21) Application number: 23910943.2
(22) Date of filing: 29.12.2023
(51) Int. Cl.: A61B 17/22, A61M 25/10

(54) **SHOCK WAVE ELECTRODE ASSEMBLY AND BALLOON CATHETER DEVICE**

(30) Priority: 29.12.2022 CN 202211718322
(71) Applicant: Shanghai Bluesail Boyuan Medical Technology Co., Ltd., Shanghai 200131 (CN)
(72) Inventor: XU, Pengfei, Shanghai 200131 (CN); CUI, Yuhu, Shanghai 200131 (CN); DUAN, Xihai, Shanghai 200131 (CN); QUE, Zhiwen, Shanghai 200131 (CN); WANG, Cheng, Shanghai 200131 (CN)
(74) Representative: Viering, Jentschura & Partner mbB Patent- und Rechtsanwälte
(86) International application number: PCT/CN2023/143297
(87) International publication number: WO 2024/141030

(57) **Abstract**

A shock wave electrode assembly (100) and a balloon catheter device (300) comprising the shock wave electrode assembly (100). The shock wave electrode assembly (100) comprises an inner electrode (110), an outer electrode (130) and an insulating layer (120), wherein the insulating layer (120) is located between the inner electrode (110) and the outer electrode (130). The inner electrode (110) is provided with a first hole (112), the insulating layer (120) is provided with a second hole (122), and the outer electrode (130) is provided with a third hole (132), wherein the diameter of the second hole (122) is not less than the diameter of the first hole (112), the diameter of the third hole (132) is not less than the diameter of the second hole (122), and the first hole (112), the second hole (122) and the third hole (132) are sequentially in communication with each other to form a cavity (140).

## Description

For all purposes, this application claims priority to Chinese Patent Application No. 202211718322.0, filed on December 29, 2022, the disclosure of which is hereby incorporated by reference in its entirety as part of this application.

### TECHNICAL FIELD

The present disclosure relates to an electrode assembly, and more particularly, to a shock wave electrode assembly and a balloon catheter device.

### BACKGROUND

Atherosclerosis is a disease with arterial stenosis and hardening caused by plaque accumulation. The plaque is composed of fibrous tissue, fat, and calcium. The accumulated calcified plaque hinders the normal flow of blood and reduces the supply of oxygen and nutrients to the body, thereby causing diseases related to the arteries supplying blood to key parts of the body (including the brain, heart and limbs).

The method of electrohydraulic effect is used to destroy the structure of the calcified lesion attached to the diseased blood vessel wall. That is, electrodes are arranged in an angioplasty-type balloon, and can be used for the operations on the calcified lesions in arterial wall by working with a high-voltage generator. In this kind of operations, the balloon catheter is advanced into the blocked area along a guide wire, and then the balloon is pressurized with conductive fluid to make the balloon pressed against the blood vessel. A series of high-voltage pulses are applied to the electrodes in the balloon by a high-voltage generator. Each pulse passes through the electrodes to generate micro bubbles in the conductive fluid. These bubbles grow and burst instantly to generate shock waves, and the shock waves travel through the balloon wall and reach the blocked area.

### SUMMARY

The present disclosure provides a shock wave electrode assembly. The shock wave electrode assembly includes: an inner electrode, an outer electrode, and an insulating layer, the insulating layer is located between the inner electrode and the outer electrode. The inner electrode is provided with a first hole, the insulating layer is provided with a second hole, the outer electrode is provided with a third hole, a diameter of the second hole is not smaller than that of the first hole, a diameter of the third hole is not smaller than that of the second hole, and the first hole, the second hole, and the third hole are sequentially communicated to form a cavity.

In an embodiment, the diameter of at least one of the second hole or the third hole is larger than that of the first hole.

In an embodiment, the diameters of the first hole, the second hole and the third hole increase sequentially, and the cavity is in a flared configuration.

In an embodiment, an axis of the cavity is perpendicular to an outer surface of the outer electrode.

In an embodiment, an axis of the cavity and an outer surface of the outer electrode intersect and are not perpendicular to each other.

In an embodiment, the inner electrode, the insulating layer, and the outer electrode are all in annular configurations and coaxially arranged, the outer electrode is sleeved outside the insulating layer, the insulating layer is sleeved outside the inner electrode, and the first hole, the second hole, and the third hole are coaxially arranged.

In an embodiment, a ratio of a thickness of the inner electrode to a thickness of the outer electrode is in a range from 1:2 to 2:1, and a ratio of a thickness of the insulating layer to the thickness of the inner electrode is in a range from 1:2 to 2:1.

In an embodiment, a ratio of the diameter of the first hole to the diameter of the second hole is in a range from 1:1 to 1:5, and a ratio of the diameter of the second hole to the diameter of the third hole is in a range from 1:1 to 1:5.

In an embodiment, a ratio of a length of the inner electrode to a length of the insulating layer is in a range from 1:2 to 1:10, and a ratio of a length of the outer electrode to a length of the insulating layer is in a range from 1:1 to 1:10.

In an embodiment, a configuration of a radial cross-section of at least one selected from the group consisting of the first hole, the second hole and the third hole is a parallelogram, a rectangle, a trapezoid, or a triangle.

In an embodiment, a configuration of at least one selected from the group consisting of the first hole, the second hole and the third hole is cylindrical, frustum-shaped, or conical.

In an embodiment, a configuration of a radial cross-section of the first hole is a parallelogram, a rectangle, a trapezoid, or a triangle; and configurations of radial cross-sections of the second hole and the third hole are each independently a parallelogram, a rectangle, or a trapezoid.

In an embodiment, the configuration of the first hole is cylindrical, frustum-shaped, or conical; and the configurations of the second hole and the third hole are each independently cylindrical or frustum-shaped.

The present disclosure provides a shock wave electrode assembly including: an inner electrode, an outer electrode, and an insulating layer, the insulating layer is located between the inner electrode and the outer electrode; the inner electrode is provided with a first hole, the insulating layer is provided with a second hole, the outer electrode is provided with a third hole, an area of a projection of the second hole along an axial direction of the second hole is not smaller than an area of a projection of the first hole along an axial direction of the first hole, an area of a projection of the third hole along an axial direction of the third hole is not smaller than the area of the projection of the second hole along the axial direction of the second hole, and the first hole, the second hole, and the third hole are sequentially communicated to form a cavity.

In an embodiment, the area of the projection of the second hole and/or the third hole along their respective axial directions is larger than the area of the projection of the first hole along the axial direction of the first hole.

In an embodiment, areas of the projections of the first hole, the second hole and the third hole along their respective axial directions increase sequentially, and the cavity is in a flared configuration.

In an embodiment, an axis of the cavity intersects an axis of the shock wave electrode assembly.

The present disclosure provides a balloon catheter device including a balloon, an inner tube, an outer tube, and at least one shock wave electrode assembly as described above; a distal end of the inner tube extends through the balloon and is connected to a distal end of the balloon, and the shock wave electrode assembly is arranged on an exterior of the inner tube located within the balloon; the outer tube is sleeved outside the inner tube, and a distal end of the outer tube is connected to a proximal end of the balloon.

In an embodiment, the balloon catheter device includes a plurality of shock wave electrode assemblies arranged at intervals along an axial direction of the inner tube; the plurality of shock wave electrode assemblies are arranged in a same circumferential direction of the inner tube or arranged at an angle along a circumferential direction of the inner tube.

In an embodiment, axial directions of the cavities of the plurality of shock wave electrode assemblies are substantially the same.

In an embodiment, the shock wave electrode assembly is provided with a plurality of cavities.

The shock wave electrode assembly according to the embodiments of the present disclosure has a cavity with a flared structure, which can reduce shock wave diffusion, focus shock waves, and increase the intensity of shock waves. Meanwhile, by changing the direction of the flared opening, the propagation direction of the shock waves can also be changed, improving the directivity of the shock waves and facilitating targeted treatment on calcified lesions and the calcified lesions within blood vessels. The inner electrode of the shock wave electrode assembly according to the embodiments of the present disclosure is an annular structure, so that a plurality of discharge cavities can be obtained without increasing the number of inner electrodes, and the annular inner electrode can also maintain good mechanical strength and is not prone to displacement during the discharge process.

### BRIEF DESCRIPTION OF DRAWINGS

In order to more clearly illustrate the technical solutions of the embodiments of the present disclosure, the drawings of the embodiments will be briefly introduced below. Obviously, the drawings in the following description only relate to some embodiments of the present disclosure and are not intended to limit the present disclosure.
Fig. 1A to Fig. 1D are schematic cross-sectional views of shock wave electrode assemblies according to some embodiments of the present disclosure.
Fig. 2 is a schematic perspective view of a shock wave electrode assembly according to an embodiment of the present disclosure.
Fig. 3 is an exploded view of the shock wave electrode assembly of Fig. 2.
Fig. 4 is a schematic diagram of the flared effect of the cavity of the shock wave electrode assembly according to an embodiment of the present disclosure.
Fig. 5 is a schematic diagram of a balloon catheter device according to an embodiment of the present disclosure.
Fig. 6A to Fig. 6C are schematic cross-sectional views of balloon catheter devices according to some embodiments of the present disclosure.
Fig. 7 is a schematic diagram of a balloon catheter device with a plurality of shock wave electrode assemblies according to some embodiments of the present disclosure.

### DETAILED DESCRIPTION

The technical solutions in the embodiments of the present disclosure will be clearly and completely described below with reference to the accompanying drawings in the embodiments of the present disclosure. Obviously, the described embodiments are only some of the embodiments of the present disclosure, rather than all of the embodiments. Based on the embodiments in present disclosure, all other embodiments obtained by those skilled in the art without making creative efforts fall within the scope of protection of present disclosure.

Unless otherwise defined, all terms (including technical and scientific terms) used in the embodiments of present disclosure have the same meaning as commonly understood by those skilled in the art to which present disclosure belongs. It should also be understood that terms such as those defined in general dictionaries should be interpreted as having meanings consistent with their meanings in the context of the related art, and should not be interpreted in an idealized or extremely formal sense, unless explicitly defined by the embodiment of the present disclosure.

The words "first", "second" and similar words used in the embodiments of present disclosure do not indicate any order, quantity or importance, but are only used to distinguish different components. Similar words such as "a", "an" or "the" do not mean a quantity limit, but mean that there is at least one. Similarly, similar words such as "include", "including", "comprise", or "comprising" mean that the elements or objects appearing before the word cover the elements or objects listed after the word and their equivalents, without excluding other elements or objects. Similar words such as "connected" or "connecting" are not limited to physical or mechanical connection, but may include electrical connection or communication connection, whether direct or indirect. The outer surface of the outer electrode" defined in the present disclosure refers to the surface of the outer electrode away from the inner electrode. The "thickness" defined in the present disclosure refers to the distance between two opposite sides of an object. In the present disclosure, the definitions of "distal end" and "proximal end" are as follows: after being inserted into a human blood vessel through the human skin, the balloon device reaches the lesion site along the direction of the human blood vessel. Taking the insertion entrance on the human skin as the reference point, along the advancing direction of the balloon, the end away from the reference point is the distal end, and the end close to the reference point is the proximal end.

Traditional angioplasty often uses a balloon catheter to physically expand lesions (such as calcified lesions) and make blood vessels unblocked again. However, when the balloon expands, it is easy to cause tearing and damage to the adventitia of the blood vessel.

The inventors of the present disclosure have found that in some existing technical solutions, the method of electrohydraulic effect is used to destroy the structure of the calcified lesion attached to the diseased blood vessel wall. That is, electrodes that are arranged in an angioplasty-type balloon, and by working with a high-voltage generator, can be used for the operations on calcified lesions in the arterial wall. However, as the discharge position of common electrode assemblies is often random, the direction of the shock waves generated by the discharge is variable, the intensity is relatively dispersed, and it is difficult to focus. To address these problems, the present disclosure provides a shock wave electrode assembly and a balloon catheter device.

The embodiments and examples of the present disclosure will be described in detail below with reference to the drawings.

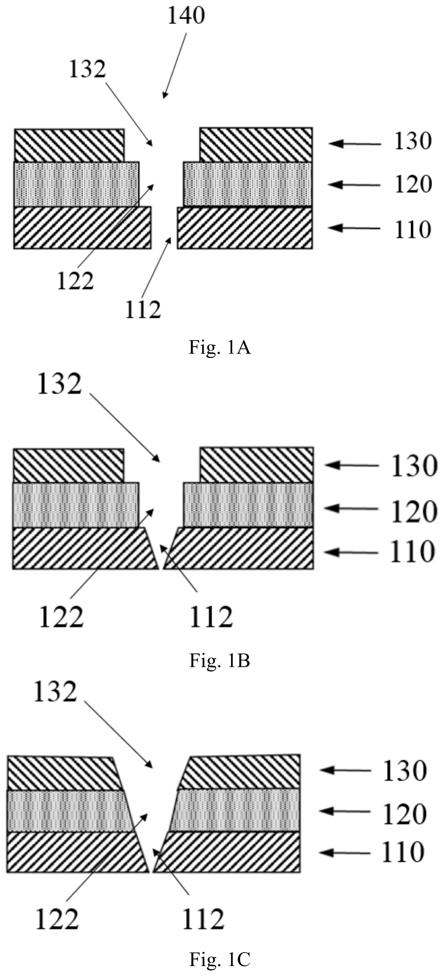

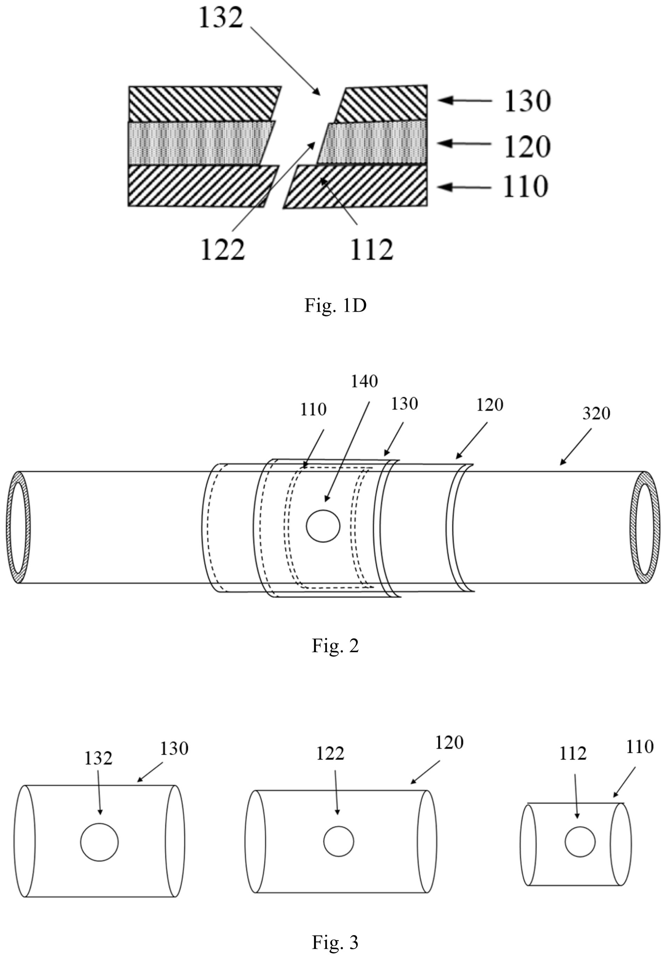

Fig. 1A to Fig. 1D are schematic cross-sectional views of shock wave electrode assemblies according to some embodiments of the present disclosure. Fig. 2 is a schematic perspective view of a shock wave electrode assembly according to an embodiment of the present disclosure. Fig. 3 is an exploded view of the shock wave electrode assembly of Fig. 2.

As shown in Fig. 1A to Fig. 1D, the shock wave electrode assembly 100 includes an inner electrode 110, an outer electrode 130, and an insulating layer 120, and the insulating layer 120 is located between the inner electrode 110 and the outer electrode 130. The inner electrode 110 is provided with a first hole 112, the insulating layer 120 is provided with a second hole 122, and the outer electrode 130 is provided with a third hole 132. The diameter of the second hole 122 is not smaller than that of the first hole 112, and the diameter of the third hole 132 is not smaller than that of the second hole 122. The first hole 112, the second hole 122 and the third hole 132 are sequentially communicated to form a cavity 140. In an embodiment, the diameter of the second hole and/or the third hole is larger than that of the first hole, and in this case, the diameter of the first hole is the smallest. In another embodiment, the diameters of the first hole, the second hole, and the third hole increase sequentially, and the cavity 140 is in a flared configuration, as shown in the figure. It can be understood that the cavity is in a flared configuration means that along the central axis direction of the cavity, the configuration of the cavity generally shows a trend of from large to small or from small to large. Thus, a certain gap is formed between the inner electrode 110 and the outer electrode 130, which facilitates the formation of a discharge circuit between the outer electrode 130 and the inner electrode 110. When the shock wave electrode assembly is placed into a liquid and given an appropriate pulse voltage, the breakdown voltage of the liquid between the inner electrode and the outer electrode can be reached, electric sparks can be emitted, and shock waves can be generated. According to the embodiments of the present disclosure, the first hole 112, the second hole 122, and the third hole 132 can be formed by any suitable physical, chemical, or physicochemical process, such as mechanical drilling, laser drilling, or masking and etching processes. It should be noted that the "the diameters... increasing sequentially" referred to in the present disclosure includes both linear changes (such as that shown in Fig. 1C) and non-linear changes of the diameter.

The present disclosure further provides a shock wave electrode assembly, which can also refer to Fig. 1A to Fig. 1D. The shock wave electrode assembly 100 includes an inner electrode 110, an outer electrode 130, and an insulating layer 120, and the insulating layer 120 is located between the inner electrode 110 and the outer electrode 130; the inner electrode 110 is provided with a first hole 112, the insulating layer 120 is provided with a second hole 122, the outer electrode 130 is provided with a third hole 132, an area of a projection of the second hole 122 along an axial direction of the second hole 122 is not smaller than an area of a projection of the first hole 112 along an axial direction of the first hole 112, an area of a projection of the third hole 132 along an axial direction of the third hole 132 is not smaller than an area of a projection of the second hole 122 along an axial direction of the second hole 122, and the first hole 112, the second hole 122 and the third hole 132 are sequentially communicated to form a cavity 140.

In an embodiment, an area of a projection of the second hole 122 and/or the third hole 132 along their respective axial directions is larger than an area of a projection of the first hole 112 along an axial direction of the first hole 112.

In an embodiment, areas of the projections of the first hole 112, the second hole 122 and the third hole 132 along their respective axial directions increase sequentially, and the cavity 140 is in a flared configuration.

In an embodiment, the axis of the cavity 140 intersects the axis of the shock wave electrode assembly 100.

According to actual needs, the direction of the shock waves can be adjusted by changing the angle of the hole(s). In an embodiment, the axis of the cavity 140 is perpendicular to the outer surface of the outer electrode 130, as shown in Fig. 1A, Fig. 1B, and Fig. 1C. In this case, the shock waves generated from the cavity 140 mainly propagate in a direction perpendicular to the outer surface of the outer electrode 130. In an embodiment, the axis of the cavity 140 intersects the outer surface of the outer electrode 130 and is not perpendicular to the outer surface of the outer electrode 130. In actual surgeries, some calcified lesions require directional treatment with shock waves. In this case, the cavity can be designed such that its axis intersects the outer surface of the outer electrode and is not perpendicular to the outer surface of the outer electrode, transforming the cavity into an inclined flared shape, as shown in Fig. 1D, so that the shock waves generated along the axis of the cavity can be directionally propagated to the calcified lesions to be treated. The shock wave electrode assembly according to the present disclosure provides the possibility of targeted treatment on calcified lesions. According to actual conditions, the angle between the axis of the cavity and the outer surface of the outer electrode can be appropriately adjusted.

In an embodiment, the inner electrode 110, the insulating layer 120 and the outer electrode 130 are all in annular configurations, as shown in Fig. 2 and Fig. 3. The inner electrode 110, the outer electrode 130, and the insulating layer 120 are coaxially arranged, the outer electrode 130 is sleeved outside the insulating layer 120 and the lower diameter of the outer electrode 130 matching the upper diameter of the insulating layer 120, thereby reducing the possibility of relative movement between the outer electrode 130 and the insulating layer 120. Similarly, the insulating layer 120 is also configured to be sleeved outside the inner electrode 110, and the lower diameter of the insulating layer 120 matches the upper diameter of the inner electrode 110, thereby ensuring the stability of the discharge process. Referring to Fig. 2, the shock wave electrode assembly including the inner electrode 110, the outer electrode 130, and the insulating layer 120 is further sleeved outside the inner tube 320. In this embodiment, the axes of the inner electrode 110, the insulating layer 120, and the outer electrode 130 are perpendicular to the axes of the first hole 112, the second hole 122 and the third hole 132.

In an embodiment, a ratio of a thickness of the inner electrode 110 to a thickness of the outer electrode 130 is in a range from 1:2 to 2:1. In an embodiment, a ratio of a thickness of the insulating layer 120 to a thickness of the inner electrode 110 is in the range from 1:2 to 2:1. According to the embodiments of the present disclosure, the insulating layer 120 mainly functions as a barrier between the inner electrode 110 and the outer electrode 130. If the thickness of the insulating layer 120 is thinner (i.e., the distance between the inner electrode 110 and the outer electrode 130 is too short), the breakdown voltage of the conductive medium between the inner electrode and the outer electrode is easily reached, generating shock waves with low energy or even causing direct conduction between the inner electrode and outer electrode with no shock wave generated. If the thickness of the insulating layer 120 is thicker (e.g., a ratio of a thickness of the insulating layer to a thickness of the inner electrode is greater than 5:1), that is, the distance between the inner electrode 110 and the outer electrode 130 is too long, the breakdown voltage of the conductive medium is difficult to reach, making it difficult to generate shock waves effectively. In the embodiments of the present disclosure, "thickness" refers to the distance between two opposite surfaces of an object. Specifically, the thickness of the inner electrode 110, the outer electrode 130, or the insulating layer 120 refers to the distance between the inner and outer surfaces at two opposite sides that are close to the inner tube 320 and away from the inner tube 320. For example, the thickness of the outer electrode 130 refers to the distance between the inner surface (which is close to the inner tube 320) and the outer surface (which is away from the inner tube 320) of the outer electrode 130. The insulating layer 120 is made of a non-conductive insulating material, such as polyimide, polyamide, fluoropolymers (e.g., PTFE, FEP, etc.), poly (ether-block-amide), polyethylene, polypropylene, and other polymers. In addition, the thickness of the insulating layer 120 is related to the dielectric constant of the insulating material. The insulating layer having high dielectric constant has good insulating performance, and under the same circumstances, the use of such relatively thinner insulating layer 120 can meet the requirements, such as using a polyamide material with a relatively high dielectric constant. Therefore, according to actual conditions, insulating materials with different dielectric constants can be used to make the insulating layer, so that the thickness ratio between the inner electrode 110, the insulating layer 120, and the outer electrode 130 will also change accordingly. In addition, the outer electrode 130 and the inner electrode 110 can be made of conductive metals, such as stainless steel, copper, silver, gold, etc.

In some embodiments, a ratio of a length of the inner electrode 110 to a length of the insulating layer 120 is in a range from 1:2 to 1:10. If the length of the inner electrode 110 is too short, the inner electrode 110 is prone to translational movement relative to the insulating layer 120. If the length of the inner electrode 110 is too long, on the one hand, it affects the delivery of the balloon catheter in the patient's body, and on the other hand, it increases material cost. Therefore, the length of the inner electrode 110 can be adjusted according to actual conditions, which is not limited in the present disclosure. In some embodiments, a ratio of a length of the outer electrode 130 to a length of the insulating layer 120 is 1:1 to 1:10. According to the embodiments of the present disclosure, once the length of the outer electrode 130 is too long, the passage convenience of the balloon catheter will be affected. Therefore, the length of the outer electrode 130 can be adjusted according to actual conditions, which is not limited in the present disclosure. According to the embodiments of the present disclosure, the length of the outer electrode 130 is smaller than that of the insulating layer 120, so that the outer electrode 130 can be better fixed over the insulating layer 120. In the embodiments of the present disclosure, the length of the inner electrode 110, the outer electrode 130, or the insulating layer 120 refers to the distance between the two opposite ends along an axial direction of the shock wave electrode assembly 100.

In an embodiment, the first hole 112, the second hole 122, and the third hole 132 are coaxially arranged, and since the insulating layer is located between the inner electrode 110 and the outer electrode 130, a certain gap is formed between the outer electrode 130 and the inner electrode 110, which is beneficial to uniform discharge. In an embodiment, a ratio of a diameter of the second hole 122 to a diameter of the third hole 132 is in a range from 1:1 to 1:5. In an embodiment, a ratio of a diameter of the first hole 112 to a diameter of the second hole 122 is in a range from 1:1 to 1:5. Due to the randomness of the discharge position of common electrode assemblies, the direction of the shock waves generated is variable and the intensity is relatively dispersed. In the shock wave electrode assembly according to the embodiments of the present disclosure, because the diameters of the first hole 112 of the inner electrode 110, the second hole 122 of the insulating layer 120, and the third hole 132 of the outer electrode 130 increase sequentially, it is ensured that the cavity 140 has a flared structure with one narrow end and one wide end. This flared structure can partially reflect diffused sound waves, focus energy, reduce diffusion loss, and superimpose wave amplitudes, thereby enabling the shock waves to increase intensityand to propagate farther.

In an embodiment, the cross-section of the first hole 112, the second hole 122, and the third hole 132 may adopt other shapes such as an ellipse in addition to the circular structure shown in the embodiments. Furthermore, the structures of the holes in the inner electrode 110, the outer electrode 130, and the insulating layer 120 may also adopt other shapes. In an embodiment, each of the configurations of radial cross-sections of the first hole 112, the second hole 122, and the third hole 132 is a parallelogram, a rectangle, a trapezoid, or a triangle.

Here, the radial cross-section refers to any cross-section where the axis of the cavity is located. In an embodiment, the configuration of the first hole 112, the second hole 122, and/or the third hole 132 is cylindrical, frustum-shaped, or conical. It is shown in Fig. 1A where the configurations of radial cross-sections of the first hole 112, the second hole 122, and the third hole 132 are all rectangles, i.e., where the configurations of the first hole 112, the second hole 122, and the third hole 132 are all cylindrical. It can be understood that a hole with a cylindrical shape may be a typical cylinder or an obliquely cut cylinder (i.e., where the axis of the cylinder is not perpendicular to the planes at both ends of the cylinder). It is shown in Fig. 1D where the configurations of radial cross-sections of the first hole 112, the second hole 122, and the third hole 132 are all parallelograms, i.e.,where the configurations of the first hole 112, the second hole 122, and the third hole 132 are all obliquely cut cylindrical. Preferably, in an embodiment, the configurations of radial cross-sections of the first hole 112 of the inner electrode 110, the second hole 122 of the insulating layer 120, and the third hole 132 of the outer electrode 130 are all trapezoids, as shown in Fig. 1C, i.e., the configurations of the first hole 112 of the inner electrode 110, the second hole 122 of the insulating layer 120, and the third hole 132 of the outer electrode 130 are all frustum-shaped. Such a structure has a more regular flared shape, which can enhance the focusing effect of the shock waves and improve the directivity and intensity of the shock waves. In principle, the length of the flared shape and the width of the flared opening have a significant impact on the focusing effect of the flared shape. A longer flared shape and a narrower opening can better display focusing effect. In some embodiments of the present disclosure, increasing the thickness of the inner electrode 110, the insulating layer and the outer electrode 130, or reducing the diameter of the third hole 132 of the outer electrode 130, can reduce the diffused propagation of shock waves, enhance the focusing effect on shock waves, and increase intensity of shock waves.

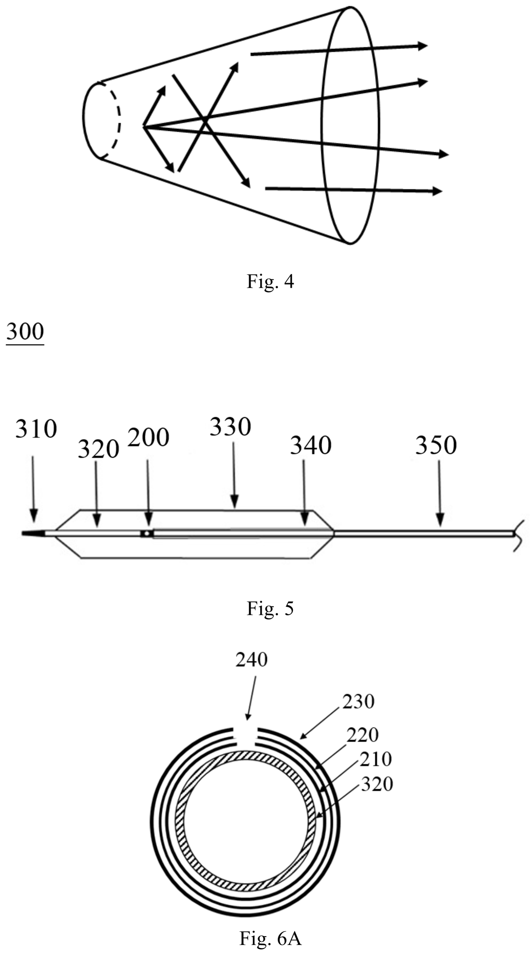

According to the embodiments of the present disclosure, a discharge circuit is formed between the outer electrode 130 and the inner electrode 110. When the shock wave electrode assembly 100 is placed in a liquid and given an appropriate pulse voltage, the breakdown voltage of the filled liquid can be reached, electric sparks can be emitted, and shock waves can be generated. The shock waves propagate through the liquid inside the balloon to impact on the balloon wall and the calcified area. Repeated pulses can disrupt the structure of calcified lesions, and expand narrowed blood vessels, without damaging surrounding soft tissues. As shown in Fig. 4, since a cavity 140 with a flared structure (narrow at one end and wide at the other end) is formed by the first hole 112 of the inner electrode 110, the second hole 122 of the insulating layer, and the third hole 132 of the outer electrode 130, this structure can partially reflect diffused sound waves, focus energy, reduce diffusion loss, and superimpose wave amplitudes, thereby enabling the shock waves to increase intensity and to propagate farther. Additionally, the direction of the shock waves can be adjusted by changing the structure of the flared shape.

Fig. 5 is a schematic diagram of a balloon catheter device according to an embodiment of the present disclosure. As shown in Fig. 5, the balloon catheter device 300 includes a distal tip 310, an inner tube 320, a balloon 330, an outer tube 350, and a shock wave electrode assembly 200. As described above, the shock wave electrode assembly 200 includes an inner electrode 210, an outer electrode 230, and an insulating layer 220, and the insulating layer 220 is located between the inner electrode 210 and the outer electrode 230. The first hole 112, the second hole 122 and the third hole 132 are respectively provided in the inner electrode 210, the insulating layer 220 and the outer electrode 230. As the detailed content of the shock wave electrode assembly has been described above, it will not be repeated here. The distal end of the inner tube 320 extends through the balloon 330 and is connected to the distal end of the balloon 330 at the distal tip 310. The shock wave electrode assembly 200 is disposed on an exterior of the inner tube 320 located inside the balloon 330; the outer tube 350 is sleeved outside the inner tube 320, and the distal end of the outer tube 350 is connected to the proximal end of the balloon 330. The interior of the inner tube 320 is a guidewire lumen for passing a guidewire through during surgery, and the gap between the inner tube 320 and the outer tube 350 forms a fluid lumen. The interior of the balloon 330 can be filled with a conductive fluid through the fluid lumen. In an embodiment, as shown in Fig. 5, the balloon catheter device 300 further includes a wire 340 extending along an axial direction of the balloon catheter device 300, and the wire 340 includes a first wire 340A and a second wire 340B. The first wire 340A is connected to the inner electrode 210, and the second wire 340B is connected to the outer electrode 230. The two wires extend along an axial direction of the balloon catheter device 300 and are connected to a high-voltage generator (not shown). Current is transmitted to the inner electrode 210 through the first wire 340A, and the inner electrode 210 forms a circuit with the outer electrode 230 through the filled conductive fluid. The high-voltage pulse provides breakdown voltage for the conductive fluid, so as to generate shock waves in the axial direction of the cavity 140. The current is then transmitted to the outer electrode 230 and returns to the high-voltage generator along the second wire 340B. The balloon catheter device according to the embodiments of the present disclosure has the above-described shock wave electrode assembly disposed inside the balloon, which can efficiently generate shock waves to rupture calcified lesions in blood vessels, effectively improving the therapeutic effect onblocked lesions. Meanwhile, the direction of the shock waves can be adjusted by changing the direction of the flared opening to achieve targeted treatment on calcified lesions.

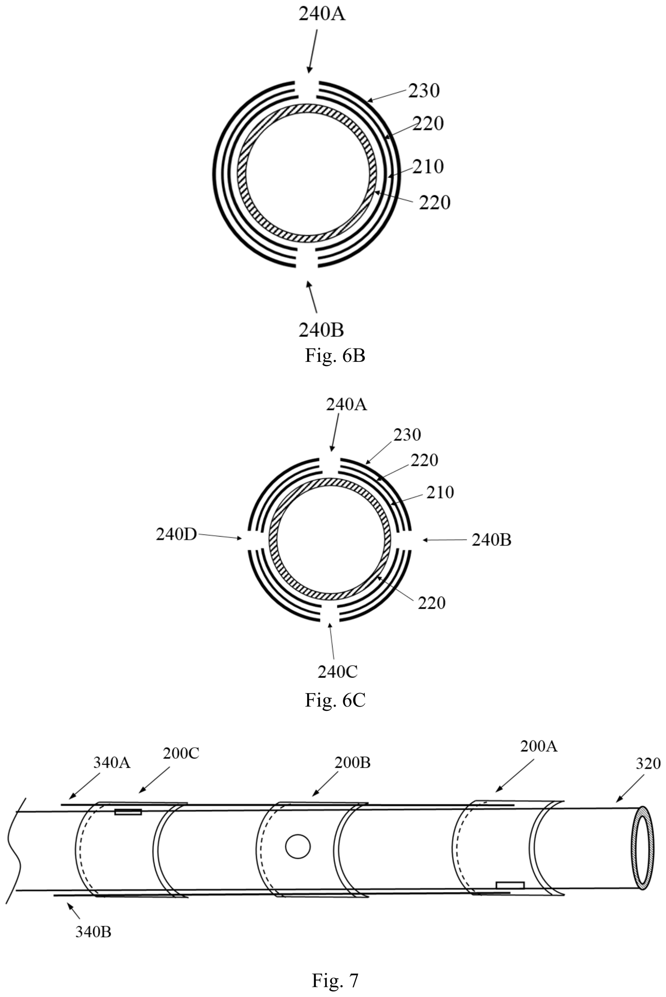

Fig. 6A is a schematic cross-sectional view of a balloon catheter device according to an embodiment of the present disclosure. The assembly process of the shock wave electrode assembly 200 in the balloon catheter device 300 will be described with reference to Fig. 3, Fig. 5, and Fig. 6A. In order to install the shock wave electrode assembly in the balloon catheter device, firstly, the inner electrode 210 is sleeved outside the inner tube 320, and the inner electrode 210 and the inner tube 320 are fixed with glue. Then, the insulating layer 220 with the second hole 222 is sleeved outside the inner electrode 210, and the insulating layer 220 is moved to align the second hole 222 with the first hole 212 of the inner electrode 210, so that the two holes are coaxial. The inner tube 320 and the insulating layer 220 are then fixed with glue. Next, the outer electrode 230 is sleeved outside, and the third hole 232 is moved to align with the second hole 222, so that the two holes are coaxial, thereby making the first hole 212, the second hole 222 and the third hole 232 coaxial and forming the cavity 140. The outer electrode 230 is then fixed on the insulating layer 220 with glue. In this embodiment, the axes of the inner electrode 210, the insulating layer 220 and the outer electrode 230 are perpendicular to the axes of the first hole 212, the second hole 222, and the third hole 232. It should be noted that because the inner tube, the inner electrode, the insulating layer, and the outer electrode all have circular cross-sections, this design reduces the difficulty of assembling the shock wave electrode assembly in the balloon catheter, simplifies the process, and facilitates the low-cost manufacturing of the balloon catheter device. In an embodiment, the shock wave electrode assembly is provided with a plurality of cavities, specifically, the inner electrode is provided with a plurality of first holes 212, the insulating layer 220 is provided with a plurality of second holes 222, and the outer electrode 230 is provided with a plurality of third holes 232. The number of the first holes 222, the number of the second holes 232, and the number of the third holes 232 are equal. Considering that in actual surgery, a plurality of calcified lesions in the circumferential direction of a blood vessel may need to be treated, if a balloon catheter device with a single shock wave electrode assembly is used, the respective calcified lesions need to be treated one by one. Therefore, in such cases, a structure with a plurality of shock wave electrode assemblies can be adopted to provide a plurality of discharge areas on the balloon catheter device, which not only enables the balloon catheter device to treat a plurality of calcified lesions simultaneously but also improves the uniformity of shock wave distribution in the circumferential space around the inner tube, thereby facilitating the treatment of calcified lesions. Fig. 6A, Fig. 6B, and Fig. 6C respectively show cross-sectional views of balloon catheter devices with shock wave electrode assemblies provided with 1, 2, or 4 cavities. Additionally, the distribution of the shock wave electrode assemblies in the circumferential direction of the inner tube 320 can be adjusted accordingly to adapt to the actual positional distribution of the calcified lesions to be treated, which is not limited in the present disclosure.

In an embodiment, the balloon catheter device 300 includes a plurality of shock wave electrode assemblies 200 arranged at intervals along an axial direction of the inner tube 320. If a plurality of calcified lesions are present in actual surgery and these lesions are spaced apart by a certain distance, it is inconvenient to transport the balloon catheter device to different calcified lesions for treatment one by one in the case where the balloon catheter device adopts a single shock wave electrode assembly 200. Therefore, in view of the above situation, a balloon catheter device with a plurality of shock wave electrode assemblies 200 arranged at intervals along an axial direction of the inner tube can be adopted. Fig. 7 shows a schematic diagram of a balloon catheter device with three shock wave electrode assemblies 200. The balloon surrounds the catheter that axially extends, and the interior of the balloon can be filled with a conductive fluid through the fluid lumen. Referring to Fig. 7, three shock wave electrode assemblies 200 (200A, 200B, and 200C) are arranged on an exterior of the inner tube 320 at certain intervals, with current transmitted through two connecting wires. The first wire 340A is connected to the inner electrodes 210A, 210B, and 210C, and the second wire 340B is connected to the outer electrodes 230A, 230B, and 230C, thereby forming a discharge circuit between the inner electrode and the outer electrode of each shock wave electrode assembly 200 to generate shock waves. Specifically, a first shock wave is generated in the axial direction of the cavity 240A of the shock wave electrode assembly 200A; a first shock wave is generated in the axial direction of the cavity 240B of the shock wave electrode assembly 200B; and a first shock wave is generated in the axial direction of the cavity 240C of the shock wave electrode assembly 200C. A similar connection method can be used to arrange a plurality of shock wave electrode assemblies inside the balloon, generating shock waves in the axial direction of the cavities of the shock wave electrode assemblies 200. Meanwhile, the distribution of different shock wave electrode assemblies 200 in the axial direction of the inner tube 320 can be adjusted according to the actual positional distribution of the calcified lesions to be treated, which is not limited in the present disclosure. In an embodiment, the axial directions of the cavities of the plurality of shock wave electrode assemblies are substantially the same. For example, for a calcified lesion area along an axial direction of the blood vessel wall, the axial directions of the cavities of the plurality of shock wave electrode assemblies arranged at intervals along an axial direction of the inner tube can be substantially uniformly directed toward the calcified lesion area, so as to perform targeted shock wave treatment on the calcified lesion area, thereby significantly improving the treatment effect.

In an embodiment, the plurality of shock wave electrode assemblies 200 are arranged in the same circumferential direction of the inner tube 320 or arranged at an angle along the circumferential direction. For a balloon catheter device provided with a plurality of shock wave electrode assemblies 200, the relative arrangement direction of the shock wave electrode assemblies 200 affects the intensity and distribution of the shock waves generated by the entire balloon. The plurality of shock wave electrode assemblies 200 can be arranged in the same circumferential direction of the inner tube 320 or arranged at an angle along the circumferential direction, according to the positions of the calcified lesions to be treated in the surgery, which is not limited in the present disclosure. Fig. 7 shows a schematic diagram of a balloon catheter device in which three shock wave electrode assemblies 200A, 200B, and 200C are arranged at 90-degree intervals along the circumferential direction of the inner tube 320, that is, the projections of the three shock wave electrode assemblies on a projection plane perpendicular to the central axis of the inner tube do not overlap. In the embodiments of the present disclosure, by using a plurality of shock wave electrode assemblies, the balloon catheter device is enabled to treat a plurality of calcified lesions simultaneously. Additionally, the positions of the plurality of shock wave electrode assemblies 200 can be arranged accordingly (e.g., in the same circumferential direction, or arranged at certain anglular intervals in sequence) according to the positions of the plurality of calcified lesions to be treated, thereby improving the treatment effect on blocked lesions, saving surgery time, and improving efficiency.

The discharge position of common electrode assemblies is usually highly random, resulting in variable directions of generated shock waves, so it is difficult to focus, and the intensity is relatively dispersed, which causes serious dissipation of shock waves during propagation and is not conducive to targeted treatment on lesions. The shock wave electrode assembly according to the embodiments of the present disclosure has a cavity with a flared structuret, which can reduce diffusion of shock waves, focus shock waves, and increase intensity of shock waves. Meanwhile, by changing the flared structure (specifically, by adjusting the direction of the flared opening), the propagation direction of the shock waves generated from the cavity of the shock wave electrode assembly can be changed. Such a design improves the directivity of the shock waves and is conducive to targeted treatment on calcified lesions and calcified lesions within blood vessels. The inner electrode of the shock wave electrode assembly according to the embodiments of the present disclosure is of an annular structure, so that a plurality of discharge cavities can be obtained without increasing the number of inner electrodes. The annular inner electrode can also maintain good mechanical strength and is not prone to displacement during the discharge process.

The following statements need to be explained.
(1) The drawings of the embodiment of present disclosure only relate to the structures involved in the embodiment of present disclosure, andcommon designs can be referred to for other structures.
(2) Without conflict, the embodiments of the present disclosure and the features in the embodiments may be combined with each other to obtain new embodiments.

The above is only the specific implementation of present disclosure, but the scope of present disclosure is not limited to this, and the scope of the present disclosure shall be subject to the scope of the claims.

## Claims

1. A shock wave electrode assembly, **characterized by** comprising:
an inner electrode, an outer electrode, and an insulating layer, wherein the insulating layer is located between the inner electrode and the outer electrode;
wherein the inner electrode is provided with a first hole,
the insulating layer is provided with a second hole,
the outer electrode is provided with a third hole,
a diameter of the second hole is not smaller than that of the first hole,
a diameter of the third hole is not smaller than that of the second hole, and
the first hole, the second hole, and the third hole are sequentially communicated to form a cavity.

2. The shock wave electrode assembly according to claim 1, **characterized in that** the diameter of at least one of the second hole or the third hole is larger than that of the first hole.

3. The shock wave electrode assembly according to claim 1 or 2, **characterized in that** the diameters of the first hole, the second hole and the third hole increase sequentially, and the cavity is in a flared configuration.

4. The shock wave electrode assembly according to any one of claims 1 to 3, **characterized in that** an axis of the cavity is perpendicular to an outer surface of the outer electrode.

5. The shock wave electrode assembly according to any one of claims 1 to 3, **characterized in that** an axis of the cavity and an outer surface of the outer electrode intersect and are not perpendicular to each other.

6. The shock wave electrode assembly according to any one of claims 1 to 5, **characterized in that** the inner electrode, the insulating layer, and the outer electrode are all in annular configurations and coaxially arranged, the outer electrode is sleeved outside the insulating layer, the insulating layer is sleeved outside the inner electrode, and the first hole, the second hole, and the third hole are coaxially arranged.

7. The shock wave electrode assembly according to any one of claims 1 to 6, **characterized in that** a ratio of a thickness of the inner electrode to a thickness of the outer electrode is in a range from 1:2 to 2:1, and a ratio of a thickness of the insulating layer to the thickness of the inner electrode is in a range from 1:2 to 2:1.

8. The shock wave electrode assembly according to any one of claims 1 to 7, **characterized in that** a ratio of the diameter of the first hole to the diameter of the second hole is in a range from 1:1 to 1:5, and a ratio of the diameter of the second hole to the diameter of the third hole is in a range from 1:1 to 1:5.

9. The shock wave electrode assembly according to any one of claims 1 to 8, **characterized in that** a ratio of a length of the inner electrode to a length of the insulating layer is in a range from 1:2 to 1:10, and a ratio of a length of the outer electrode to a length of the insulating layer is in a range from 1:1 to 1:10.

10. The shock wave electrode assembly according to any one of claims 1 to 9, **characterized in that** a configuration of a radial cross-section of at least one selected from the group consisting of the first hole, the second hole and the third hole is a parallelogram, a rectangle, a trapezoid, or a triangle.

11. The shock wave electrode assembly according to any one of claims 1 to 9, **characterized in that** a configuration of at least one selected from the group consisting of the first hole, the second hole and the third hole is cylindrical, frustum-shaped, or conical.

12. A shock wave electrode assembly, **characterized by** comprising:
an inner electrode, an outer electrode, and an insulating layer, wherein the insulating layer is located between the inner electrode and the outer electrode;
wherein the inner electrode is provided with a first hole,
the insulating layer is provided with a second hole,
the outer electrode is provided with a third hole,
an area of a projection of the second hole along an axial direction of the second hole is not smaller than an area of a projection of the first hole along an axial direction of the first hole,
an area of a projection of the third hole along an axial direction of the third hole is not smaller than the area of the projection of the second hole along the axial direction of the second hole, and
the first hole, the second hole, and the third hole are sequentially communicated to form a cavity.

13. The shock wave electrode assembly according to claim 12, **characterized in that** the area of the projection of the second hole and/or the third hole along their respective axial directions is larger than the area of the projection of the first hole along the axial direction of the first hole.

14. The shock wave electrode assembly according to claim 12, **characterized in that** areas of the projections of the first hole, the second hole and the third hole along their respective axial directions increase sequentially, and the cavity is in a flared configuration.

15. The shock wave electrode assembly according to any one of claims 12 to 14, **characterized in that** an axis of the cavity intersects an axis of the shock wave electrode assembly.

16. A balloon catheter device, **characterized by** comprising a balloon, an inner tube, an outer tube, and at least one shock wave electrode assembly according to any one of claims 1 to 15; a distal end of the inner tube extends through the balloon and is connected to a distal end of the balloon, and the shock wave electrode assembly is arranged on an exterior of the inner tube located within the balloon; the outer tube is sleeved outside the inner tube, and a distal end of the outer tube is connected to a proximal end of the balloon.

17. The balloon catheter device according to claim 16, **characterized in that** the balloon catheter device comprises a plurality of shock wave electrode assemblies arranged at intervals along an axial direction of the inner tube; the plurality of shock wave electrode assemblies are arranged in a same circumferential direction of the inner tube or arranged at an angle along a circumferential direction of the inner tube.

18. The balloon catheter device according to claim 17, **characterized in that** axial directions of the cavities of the plurality of shock wave electrode assemblies are substantially the same.

19. The balloon catheter device according to claim 18, **characterized in that** the shock wave electrode assembly is provided with a plurality of cavities.
